# EUROPÄISCHE PATENTSCHRIFT

(11) **EP 0 431 378 B1**
(45) Veröffentlichungstag und Bekanntmachung des Hinweises auf die Patenterteilung: **12.01.1994**
(21) Anmeldenummer: 90122008.7
(22) Anmeldetag: 17.11.1990
(51) Int. Cl.: B28C 7/04, G01N 5/00, G01N 33/38, G01N 9/02

(54) **Verfahren zur Feuchtemessung von Schüttgütern bei der chargenweisen Herstellung von Baustoffmischungen**
Method for measuring moisture content of bulk materials in the batch production of building material compositions
Procédé de mesure d'humidité de matériaux en vrac pour la préparation par lots de matériaux de construction

(30) Priorität: 02.12.1989 DE 3939902
(43) Veröffentlichungstag der Anmeldung: 12.06.1991
(73) Patentinhaber: ELBA-WERK Maschinen-Gesellschaft mbH, D-76275 Ettlingen (DE)
(72) Erfinder: Bittmann, Peter, W-7560 Gaggenau (DE); Huber, Helmut, W-7601 Schutterwald (DE); Kiefer, Paul, W-7601 Schutterwald (DE); Benz, Erhard, W-7611 Berghaupten (DE)
(74) Vertreter: Zahn, Roland, Dipl.-Ing.

(56) Entgegenhaltungen:
- EP-A- 0 276 882
- DE-B- 1 112 440
- DE-C- 687 184
- FR-A- 2 401 005
- US-A- 3 186 596

## Beschreibung

Die vorliegende Erfindung bezieht sich auf ein Verfahren zur Bestimmung der Eigenfeuchte der jeweils einem Materialstrom zugeordneten Zuschlagstoff-Fraktion bei der chargenweisen Herstellung von Baustoffmischungen, insbesondere Betonmischungen, wobei das zu jeder Zuschlagstoff-Fraktion gehörende, einem bestimmten Volumen zugeordnete Trockengewicht als Referenzgröße für die aktuelle Zuschlagstoff-Fraktion dient.

Die Güteeigenschaften von Baustoffmischungen, insbesondere Beton, sind ganz wesentlich bestimmt durch den sogenannten Wasser-/Zement-Wert. Nur wenn dieser Wasser-/Zement-Wert für die jeweils gewünschte Baustoffmischung der vorgegebenen Rezeptur entsprechend genau und jederzeit reproduzierbar vorliegt bzw. gewährleistet ist, hat die Baustoffmischung auch die gewünschten vorbestimmten Eigenschaften. Angesichts dessen, daß die Baustoff- - insbesondere die Betonbereitung - heutzutage völlig automatisiert abläuft, kommt der Integration der Feuchtebestimmung der Zuschlagstoff-Fraktionen in den Verfahrensablauf insofern eine besondere Bedeutung bei, als eben diese Feuchtebestimmung ebenfalls automatisch erfolgen und Berücksichtigung finden muß. Ausgangspunkt dieser Be- trachtungen und Überlegungen ist der Umstand, daß die Zuschlagstoff-Fraktionen in sich und gegebenenfalls relativ zueinander eine unterschiedliche Eigenfeuchte aufweisen, die letztlich bei der Herstellung einer Baustoffmischung zu berücksichtigen wäre - und zwar insoweit, als die über die Eigenfeuchte vorhandene Wassermenge bei der Zugabe der rezepturabhängigen Wassermenge in Abzug zu bringen ist.

Nach dem Stand der Technik sind zur Bestimmung der (Eigen-)Feuchte von Schüttgütern bzw. Zuschlagstoffen bei der Betonbereitung unterschiedliche, mehr oder weniger aufwendige und komplizierte Meßverfahren und Meßgeräte bekannt, Es sind dies z. B. Leitfähigkeitsmeßgeräte, Dielektrische Feuchtemeßgeräte (DEOS 36 12 282), Kapazitive Feuchtemeßgeräte, Mikrowellen-Feuchtemeßgeräte, Kernphysikalische Verfahren, Optische Feuchtemeßgeräte, Infrarotmessungen an Feststoffen, Thermische Feuchtemeßgeräte, Ultraschallmeßgeräte, Kalorimetrische Verfahren, Hygroskopische Gleichgewichts-Verfahren, und Feuchteausgleichsverfahren.

Aus der AT-PS 2 05 900 ist bereits ein Verfahren zur Herstellung von Betonmischungen unter kontinuierlicher Berücksichtigung der Eigenfeuchtigkeit der Betonzuschlagstoffe bei jedem einzelnen Mischvorgang bekannt, gemäß dem jeweils das zu einer Fraktion gehörende Trockengewicht als Vergleichsmaß für die jeweils aktuellen Zuschlagstoff-Fraktionen benutzt wird. Beim Dosieren der Zuschlagstoffe wird jeweils eine Teilmenge konstanten Volumens in eine separate Wägevorrichtung abgezweigt und verwogen. Aus dem Vergleich des Trockengewichts einerseits und des Gewichts der abgezweigten Teilmenge andererseits wird die der Eigenfeuchte der gesamten dosierten Zuschlagstoff-Fraktion entsprechende Wassermenge hochgerechnet und bei der Zugabe des sogenannten Anmachwassers berücksichtigt.

Der Nachteil bzw. die Unzulänglichkeit dieses aus dem Stand der Technik bekannten Verfahrens und der oben genannten weiteren Verfahren bzw. Feuchtemeßgeräte besteht darin, daß jeweils nur quasi stichprobenweise Teilbereiche bzw. Teilmengen einer dosierten Zuschlagstoff-Fraktion auf ihre Eigenfeuchte hin "vermessen" werden. Da diese Eigenfeuchte jedoch über die Gesamtmenge bzw. das Gesamtvolumen einer dosierenden Zuschlagstoff-Fraktion betrachtet zum Teil ganz erhebliche Unterschiede aufweisen kann, sind die Stichprobenergebnisse" nicht geeignet, die gesamte Eigenfeuchte und damit die gesamte in der Zuschlagstoff-Fraktion gebundene Wassermenge mit der erforderlichen Genauigkeit zu ermitteln. Mit den bekannten Verfahren und Vorrichtungen erhält man letztlich nur ein Teilergebnis aus der Gesamtheit der Zuschlagstoff-Fraktion und nicht, wie es für eine wirklich genaue Bestimmung des Anmachwassers erforderlich ware. ein sogenanntes repräsentatives Ergebnis für die gesamte Zuschlagstoff-Fraktion.

Die der vorliegenden Erfindung zugrunde liegende Aufgabe besteht darin, ein Verfahren der gattungsgemäßen Art anzugeben, mit dem für die Gesamtmenge einer dosierten Zuschlagstoff- Fraktion die Eigenfeuchte bestimmt wird und zwar unmittelbar, d. h. ohne extrapolatorische Berechnungen auf der Grundlage von Teil-Meßergebnissen.

Diese Aufgabe wird dadurch gelöst, daß in Verbindung mit einem der jeweiligen Zuschlagstoff-Fraktion zugeordneten konstanten Volumenstrom der Massendurchsatz für die völlig trockene Zuschlagstoff-Fraktion ermittelt wird, und daß die für eine bestimmte Baustoffmischung erforderliche jeweilige Menge der jeweiligen feuchten Zuschlagstoff-Fraktion die dem genannten, einer bestimmten Dosierzeit zugehörigen, zeitlich konstanten Volumenstrom zugeordnet ist, entnommen und verwogen wird, so daß aus dem Massendurchsatz der jeweiligen völlig trockenen Zuschlagstoff-Fraktion, der jeweiligen Dosierzeit und dieser jeweiligen Menge die Eigenfeuchte der Zuschlagstoff-Fraktion bestimmbar ist.

Letztlich ist es dabei unerheblich, ob die erforderliche Gesamtmenge Anmachwasser jeweils nach der Verwiegung einer Zuschlagstoff-Fraktion oder nach der Verwiegung aller Zuschlagstoff-Fraktionen - unter Zwischenspeicherung der Ergebnisse aller Zuschlagstoff-Fraktiönen - ermittelt wird.

Die besonderen Vorteile dieser Verfahrensweise liegen darin, daß wirklich die gesamte Menge des an die Partikel der Zuschlagstoff-Fraktionen gebundenen Wassers ermittelt wird, und daß dieses Ergebnis letztlich mit ganz einfachen Mitteln erzielt wird. Schließlich ist davon auszugehen, daß nur die Eigenschaften der ohnehin vorhandenen und erforderlichen Waage der Betonbereitungsanlage konsequent genutzt werden

Das erfindungsgemäße Verfahren wird im folgenden nochmals etwas ausführlicher erläutert.

Voraussetzung für das Funktionieren, sowie die Genauigkeit und Reproduzierbarkeit des Verfahrens ist, daß quasi in einer Vorstufe zum eigentlichen Verfahren für die bzw. sämtliche in Frage kommenden Zuschlagstoff-Fraktionen ein Referenzwert für einen Massendurchsatz ermittelt wird. Dieser Referenzwert wird als Meßgröße der in einer Zeiteinheit volumetrisch dosierten Masse der völlig trockenen Zuschlagstoff-Fraktion bestimmt und abgespeichert. Die volumetrische Dosierung selbst erfolgt mittels geeigneter und an sich bekannter Dosierorgane, wie z.B. Förderbändern, denen zur Gewährleistung eines stets gleichbleibenden Materialquerschnitts und damit eines stets gleichbleibenden Volumens eine komplementäre Abstreifvorrichtung zugeordnet ist.

Bei der Herstellung einer Baustoffmischung, wie z.B. einer Betonmischung, werden nun nacheinander die einzelnen Zuschlagstoff-Fraktionen jeweils separat für sich in die in der Betonbereitungsanlage ohnehin vorhandene und erforderliche Waage eindosiert, und zwar gleichermaßen volumetrisch wie bei der Ermittlung der Referenzwerte. Ist der Dosiervorgang jeweils beendet, so wird die in die Waage eindosierte Menge verwogen.

Dieses Wägeergebnis wird nun mit dem der trockenen Zuschlagstoff-Fraktion entsprechenen Referenzwert in Korrelation gebracht, und zwar wird über den Referenzwert und die Dosierzeit der dosierten Zuschlagstoff-Fraktion ein "theoretischer" Gewichtswert bestimmt, der dem Gewicht der Zuschlagstoff-Fraktion entspräche, wenn diese völlig trocken wäre.

Die Differenz zwischen dem mit der Waage ermittelten Gewichtswert und dem genannten theoretischen Gewichtswert entspricht nunmehr der Eigenfeuchte, d. h. der Wassermenge, die in der Zuschlagstoff-Fraktion und in Summe in der Gesamtheit der Zuschlagstoff-Fraktionen gebunden ist.

Bei der Festlegung der Gesamtmenge des der Baustoffmischung zuzugebenden Anmachwassers wird nun die rechnerisch ermittelte Menge bereits vorhandenen Wassers in Abzug gebracht, und zwar als nunmehr wirklich repräsentative Wert für die jeweils gesamte Zuschlagstoff-Fraktion.

## Patentansprüche

1. Verfahren zur Bestimmung der Eigenfeuchte der jeweils einem Materialstrom zugeordneten Zuschlagstoff-Fraktion bei der chargenweisen Herstellung von Baustoffmischungen, insbesondere Betonmischungen,
wobei das zu jeder Zuschlagstoff-Fraktion gehörende, einem bestimmten Volumen zugeordnete Trockengewicht als Referenzgröße für die aktuelle Zuschlagstoff-Fraktion dient, **dadurch gekennzeichnet,**
daß in Verbindung mit einem der jeweiiigen Zuschlagstoff-Fraktion zugeordneten konstanten Volumenstrom der Massendurchsatz für die völlig trockene Zuschlagstoff-Fraktion ermittelt wird, und
daß die für eine bestimmte Baustoffmischung erforderliche jeweilige Menge der jeweiligen feuchten Zuschlagstoff-Fraktion die dem gesamten, einer bestimmten Dosierzeit zugehörigen, zeitlich konstanten Voiumenstrom zugeordnet ist, entnommen und verwogen wird,
so daß aus dem Massendurchsatz der jeweiligen völlig trockenen Zuschlagstoff-Fraktion, der jeweiligen Dosierzeit und dieser jeweiligen Menge die Eigenfeuchte der Zuschlagstoff-Fraktion bestimmbar ist.

## Claims

1. Method for determination the inherent moisture content of an individual aggregate material fraction, being arranged at times within a material stream flow, in the batch production of building material compositions especially of concrete mixtures,
- whereby the dry weight of a certain volume belonging to an individual aggregate fraction serves as reference size for the actual aggregate fraction
characterized by that,
- that in connection with a constant volume flow associated with each respective individual aggregate fraction, the mass through-passing for the entirely dry aggregate fraction will be determinated and
- that the respective quantity, necessary for a certain building material mixture of the actual moisten aggregate fraction, associated to the total timely constant volume flow within assigned batching time, will be extracted and weighed
- so that from the mass through-passing of the entire dry aggregate fraction, the assigned batching time and of the respective quantity the inherent moisture content of the aggregate fraction can be determined.

## Revendications

1. Procédé pour la détermination de l'humidité propre de chaque fraction d'agrégats composant un flux de matériaux lors de la fabrication, par gâchées successives, de mélanges de matériaux de construction, notamment de béton,
- où le poids à sec d'un volume déterminé de matériaux appartenant à chaque fraction d' aggrégats sert de dimension de référence pour la fraction d'agrégates concernée
se caractérisant par le fait
- que l'on détermine, en relation avec un flux d'écoulement constant pour chaque fraction d'agrégat considérée, la masse écoulée pour la fraction d'agrégats parfaitement sèche et
- que l'on prélève et pèse la quantité respective de chaque fraction de matériaux humide nécessaire à la fabrication d'un certain type de mélange composant le flux total d'écoulement constant correspondant à un temps de dosage donné
- de telle manière qu'à partir de la masse de matériaux parfaitement sèche de la fraction d'aggrégats considérée écoulée, du temps de dosage et de la quantité correspondante, l'humidité propre de la fraction de matériaux puisse être déterminée.
